(19) Europäisches Patentamt

European Patent Office

Office européen des brevets

(11) **EP 0 922 688 A1**

(12) **EUROPÄISCHE PATENTANMELDUNG**

(43) Veröffentlichungstag:
16.06.1999 Patentblatt 1999/24

(51) Int. Cl.$^6$: **C07C 29/149**, B01J 23/76

(21) Anmeldenummer: 98122726.7

(22) Anmeldetag: 28.11.1998

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 10.12.1997 DE 19754788

(71) Anmelder: BAYER AG
51368 Leverkusen (DE)

(72) Erfinder:
• Darsow, Gerhard, Dr.
47804 Krefeld (DE)
• Dummer, Wolfgang, Dr.
47800 Krefeld (DE)
• Niemeier, Wilfried, Dr.
47803 Krefeld (DE)

(54) **Verfahren zur Herstellung von aliphatischen Alpha, omega-Diolen**

(57) In einem Verfahren zur Herstellung von aliphatischen $\alpha,\omega$-Diolen mit 4 bis 12 C-Atomen aus aliphatischen $\alpha,\omega$-Dicarbonsäuren mit 4 bis 12 C-Atomen wird zunächst aus Diol und Dicarbonsäure ein Oligoester der mittleren Kettenlänge n = 3 bis n = 4,5 und Säurezahlen von 40 bis 60 mg KOH/g Reaktionsgemisch gebildet, der danach katalytisch in der Flüssigphase hydriert wird. Die Hydrierung wird kontinuierlich bei 180 bis 250°C und einem $H_2$-Druck von 100 bis 400 an einem Zn-Oxid-freien stückigen Katalysator aus verpreßten Pulvern von Cu-, Mn- und Al-Oxiden mit einem Gehalt von mindestens einem Oxid von Metallen der VI. Nebengruppe des Periodensystems der Elemente (Mendelejew) durchgeführt. Die $H_2$-Menge beträgt das 20- bis 100-fache der stöchiometrisch nötigen Menge.

EP 0 922 688 A1

**Beschreibung**

Die vorliegende Erfindung betrifft ein vereinfachtes Verfahren zur Herstellung von aliphatischen $\alpha,\omega$-Diolen mit 4 bis 12 C-Atomen aus aliphatischen $\alpha,\omega$-Dicarbonsäuren mit 4 bis 12 C-Atomen, bei welchem nur sehr geringe Mengen der üblicher-weise bei der Hydrierung solcher Säuren oder ihrer Esterderivate als Nebenprodukt entstehenden Monoalkohole der C-Zahlen 1 bis 12 sowie keine $C_4$-$C_{12}$-Lactone und keine $C_4$-$C_{12}$-$\omega$-Hydroxycarbonsäuren gebildet werden. Hierzu wird auf den Einsatz von Monoalkoholen für die Esterherstellung verzichtet, indem man zunächst aus der einzusetzenden Carbonsäure und dem herzustellenden Diol ein oligomeres Diol-Dicarboxylat mit definierter mittlerer Kettenlänge (durchschnittliche Esterlänge = Oligomerisierungsgrad n = 3 bis n = 4,5) und Säurezahlen von 40 bis 60 mg KOH/g Reaktionsgemisch herstellt, das anschließend ohne weitere destillative Reinigung über neu entwickelten ZnO-freien stückigen Katalysatoren aus verpreßten Pulvern von Cu-, Mn- und Al-Oxiden mit einem Gehalt von mindestens einem Oxid von Metallen der VI. Nebengruppe des Periodensystems der Elemente (Mendelejew), insbesondere Cr, Mo, W, in der Flüssigphase mit Wasserstoff hydriert wird. Die genannten Umstände machen das erfindungsgemäße Verfahren ökologisch und technisch vorteilhaft.

Aliphatische $\alpha,\omega$-Diole, beispielsweise Hexandiol-1,6, sind wichtige Monomere für die Produktion von thermoplastischen Polyestern sowie von Polyurethanen mit besonderen mechanischen und chemischen Eigenschaften. Hierzu können solche Diole in reiner Form oder als Gemische mehrerer Diole mit unterschiedlichen Kettenlängen im $C_4$-$C_{12}$-Bereich eingesetzt werden.

Zur Herstellung beispielsweise von Hexandiol-1,6 ist es bekannt, Adipinsäure oder ihre Salze direkt in wäßriger Lösung oder in einem organischen Lösungsmittel dikontinuierlich (DE-OS 26 05 107; GB 1.300.889) oder kontinuierlich (DE-OS 23 21 101) zu hydrieren, wobei neben Hexandiol-1,6 stets größere Anteile an Caprolacton, $\omega$-Hydroxycapronsäure sowie Monoalkohole der C-Zahlen 1 bis 6 gebildet werden. Zum gleichen Zweck ist es weiterhin bekannt, Adipinsäure mit Monoalkoholen zu einem Di-n-alkyladipinat zu verestern und dieses in der Gasphase zu Hexandiol-1,6 zu hydrieren (WO 82/03854). Es ist außerdem bekannt, Adipinsäure diskontinuierlich mit Diolen, wie Hexandiol-1,6 zu einem Gemisch höherer Ester mit der Säurezahl 10 bis 15 mg KOH/g Reaktionsprodukt zu verestern und dieses in einem diskontinuierlichen Autoklavenprozeß zu Hexandiol-1,6 und $\omega$-Hydroxy-capronsäureester zu hydrieren, wobei als Hydrierkatalysator ein pulverförmiger Kupferchromit-Katalysator zum Einsatz gelangt (DE-AS 10 23 750).

Aus EP-A 721 928 ist ein Verfahren zur Herstellung von aliphatischen $\alpha,\omega$-Diolen mit 4 bis 12 C-Atomen aus aliphatischen $\alpha,\omega$-Dicarbonsäuren mit 4 bis 12 C-Atomen durch Oligoveresterung der Dicarbonsäuren mit den Diolen und katalytische Hydrierung des entstandenen Oligoesters in der Flüssigphase bekannt, das dadurch gekennzeichnet ist, daß der Oligoester kontinuierlich an einem stückigen Katalysator, bestehend aus verpreßten Pulvern von Cu-, Zn- und Al-Oxiden mit einem oder ohne einen Gehalt von mindestens einem Oxid von Metallen der Eisengruppe des Periodensystems der Elemente (Mendelejew) oder des Mangans, insbesondere Fe, Cr, Ni, Mn, hydriert wird. Es zeichnet sich weiterhin dadurch aus, daß die Oligoveresterung diskontinuierlich oder quasikontinuierlich in 1 bis 4 Veresterungsstufen, bevorzugt quasi-kontinuierlich in 2 oder 3 Veresterungsstufen unter destillativer Entfernung des Reaktionswassers durchgeführt werden muß und daß der eingesetzte Oligoester so hergestellt werden muß, daß eine Säurezahl von maximal 50 mg KOH/g Oligoester (bei oligomerem Hexandiol-1,6-adipinat maximal 32 mg KOH/g Oligoester) eingehalten wird. In den Versuchsbeispielen wurden ausschließlich Apparaturen mit je drei Veresterungsstufen beschrieben. Ein solches Verfahren erscheint aus heutiger Sicht äußerst umständlich und kostspielig.

Es war daher völlig unerwartet und überraschend, daß beim Einsatz der neuen besonders für die Hydrierung von Oligoestern geeigneten Katalysatoren, diese mehrstufigen Veresterungsapparaturen nicht mehr notwendig sind, weil die Veresterung mit den zulässigen Säurezahlen von 40 bis 60 mg KOH/g Reaktionsgemisch kurzzeitig in einem einzigen Veresterungsschritt durchgeführt werden kann. Dieser Verfahrensfortschritt ließ sich deshalb erzielen, weil die neuen Hydrierkatalysatoren erheblich höhere Säurezahlen bei den zu hydrierenden Oligoestern vertragen als bisher.

Die Erfindung betrifft somit ein Verfahren zur Herstellung von aliphatischen $\alpha,\omega$-Diolen mit 4 bis 12 C-Atomen aus aliphatischen $\alpha,\omega$-Dicarbonsäuren mit 4 bis 12 C-Atomen durch Oligoveresterung der genannten Dicarbonsäuren mit den genannten Diolen in einem Reaktionsschritt und katalytische Hydrierung des entstandenen Oligoesters in der Flüssigphase, das dadurch gekennzeichnet ist, daß ein Oligoester mit Säurezahlen von 40 bis 60 mg KOH/g kontinuierlich bei 180 bis 250°C, bevorzugt 190 bis 240°C, und einem $H_2$-Druck von 100 bis 400 bar, bevorzugt von 150 bis 300 bar, wobei die $H_2$-Menge das 20- bis 100-fache der stöchiometrisch nötigen Menge beträgt, an einem Zn-Oxidfreien stückigen Katalysator, bestehend aus verpreßten Pulvern von Cu-, Mn- und Al-Oxiden mit einem Gehalt von mindestens einem Oxid von Metallen der VI. Nebengruppe des Periodensystems der Elemente (Mendelejew), insbesondere Cr, Mo, W, hydriert wird.

Der Reaktionsverlauf läßt sich anhand der Bildung von Hexandiol-1,6 aus Adipinsäure unter Benutzung von Hexandiol-1,6 zur Oligoveresterung durch das folgende Reaktionsschemas veranschaulichen:

$$n \text{ HOOC - } (CH_2)_4 \text{ - COOH} + (n + 1) \text{ HO - } (CH_2)_6 \text{ - OH} \xrightarrow{-2n\ H_2O}$$

EP 0 922 688 A1

$$HO - (CH_2)_6 - [O - CO - (CH_2)_4 - CO - O - (CH_2)_6]_n - OH \xrightarrow[\text{(Kat.)}]{-4n\,H_2}$$

$$(2n + 1)\; HO - (CH_2)_6 - OH$$

In den bisher bekanntgewordenen Verfahren treten häufig größere Mengen an Nebenprodukten auf, die einen erheblichen Aufwand bei der Reindarstellung des Reaktionsproduktes verursachen. Eine Recyclisierung ist nicht immer möglich, da die Hydrierung zum gewünschten Endprodukt, wie oben bereits erwähnt, nicht immer glatt verläuft und daher zu einem höheren Niveau an im Kreis geführten Nebenprodukten führen würde. Eine grundsätzliche Schwierigkeit bereitet ferner der zur Veresterung bislang eingesetzte Monoalkohol, der vom Endprodukt abgetrennt werden muß. Das Festhalten an Monoalkoholen zur Veresterung erklärt sich ganz offenbar aus der Tatsache, daß bei der Fortentwicklung des Hydrierverfahrens von der flüssigen zur gasförmigen Phase ein leicht verdampfbarer Ester zur Verfügung stehen mußte. Für solche leicht verdampfbaren Ester aus Monoalkoholen spricht ferner die Tatsache, daß sie in übersichtlicher Weise rein dargestellt werden können; dies schien sehr wichtig zu sein, da bei Hydrierungen verunreinigte Ausgangsmaterialien in der Regel zu Schwierigkeiten führen. Solche Ester aus Dicarbonsäuren und niederen Alkoholen, beispielsweise Methanol, Ethanol oder Propanol, erfordern in der Regel die Benutzung eines Veresterungskatalysators und eines Wasserschleppmittels, wie beispielsweise Toluol, die beide weitere systemfremde Stoffe darstellen, die abgetrennt werden müssen. Die Benutzung eines Wasserschleppmittels, das als azeotropes Gemisch mit dem gebildeten Reaktionswasser aus dem Reaktionsgemisch aodestilliert, vom Wasser abgetrennt und wieder in das Reaktionsgemisch zurückgeführt werden muß, bedeutet einen zusätzlichen apparativen Aufwand. Weiterhin ist verhältnismäßig viel Energie aufzuwenden, um das Schleppmittel, dessen Menge ein Vielfaches der gebildeten Wassermenge beträgt, wiederholt aus dem Reaktionsgemisch abzudestillieren. Schließlich muß der Dicarbonsäure-Di-alkylester vor der katalytischen Hydrierung durch Destillation oder andere umständliche Reinigungsmaßnahmen sorgfältig vom Veresterungskatalysator befreit werden. Nach der Hydrierung muß der Monoalkohol mit technischem Aufwand von den Reaktionsprodukten getrennt, auf reinen Alkohol aufgearbeitet und wieder dem Veresterungsprozeß zugeführt werden, wobei nicht zu vermeidende Alkoholverluste ergänzt werden müssen.

Wollte man eine Dicarbonsäure mit höhersiedenden Alkoholen, beispielsweise solchen mit C-Zahlen von >3, verestern, um sie als Dialkylester in der Gasphase zu hydrieren, kann man möglicherweise auf den Veresterungskatalysator und das Schleppmittel verzichten, benötigt aber weiterhin als Reaktionsfremden Stoff den relativ teuren, höhersiedenden Monoalkohol, der ebenfalls wieder auf reinen Alkohol aufgearbeitet und wieder dem Veresterungsprozeß zugeführt werden muß, wobei auch hier nicht zu vermeidende Alkoholverluste ergänzt werden müssen, die wegen des höheren Preises solcher Alkohole die Wirtschaftlichkeit eines solchen Verfahrens empfindlich treffen. Wegen der höheren Siedepunkte ist zusätzlich jeder Destillationsaufwand größer.

Anders und vorteilhafter liegt der Fall, wenn zur Veresterung der Dicarbonsäure mit 4 bis 12 C-Atomen ein Diol mit 4 bis 12 C-Atomen eingesetzt wird, da in einem solchen Fall weder Veresterungskatalysatoren noch Schleppmittel erforderlich sind und da das Veresterungsdiol als systemeigener Stoff im Reaktionsprodukt verbleiben kann. Für die obengenannten Verwendungszwecke ist es in vielen Fällen zulässig, ein Gemisch von Diolen mit verschiedener Kettenlänge einzusetzen; dadurch ist es nicht erforderlich, zur Oligoveresterung der Dicarbonsäure ein Diol mit der gleichen Anzahl von C-Atomen einzusetzen. Vielmehr ist es erfindungsgemäß zulässig, beispielsweise Adipinsäure außer mit Hexandiol-1,6 auch mit Butandiol-1,4, Octandiol-1,8 und anderen Diolen umzusetzen, um dann z.B. ein Gemisch von beispielsweise $C_6$-Diol mit $C_8$-Diol zu erhalten. Um jedoch $\alpha,\omega$-Diole als Reaktionsprodukte mit einheitlicher C-Zahl zu erhalten, wird die umzusetzende Dicarbonsäure mit einem Diol gleicher C-Zahl oligoverestert und hydriert, beispielsweise Adipinsäure mit Hexandiol-1,6, Bernsteinsäure mit Butandiol-1,4, Suberinsäure (Korksäure) mit Octandiol-1,8 usw.

Erfindungsgemäß einsetzbare Dicarbonsäuren mit geradzahligen oder ungeradzahligen C-Atomen, wobei die C-Atome der beiden Carboxylgruppen stets mitgezählt werden, sind beispielsweise: Bernsteinsäure, Glutarsäure, Adipinsäure, Pimelinsäure, Suberinsäure (Korksäure), Heptandicarbonsäure, Octandicarbonsäure und Decandicarbonsäure. Sie sind bekannt und aus natürlichen oder synthetischen Quellen zugänglich.

Erfindungsgemäß herzustellende und im Schritt der Oligoveresterung einsetzbare $\alpha,\omega$-Diole mit geradzahligen oder ungeradzahligen C-Atomen sind beispielsweise Butandiol-1,4, Pentandiol-1,5, Hexandiol-1,6, Heptandiol-1,7, Octandiol-1,8, Decandiol-1,10 und Dodecandiol-1,12.

Das erfindungsgemäße Verfahren ist besonders wichtig für die Herstellung von Hexandiol-1,6 durch Oligoveresterung von Adipinsäure mit Hexandiol-1,6 und anschließende Hydrierung.

Zur Herstellung von Oligoestern, wie sie im erfindungsgemäßen Verfahren benötigt werden, beispielsweise zur Herstellung von Hexandiol-1,6-adipinat, wird normalerweise ein diskontinuierliches Verfahren (Batch-Prozeß) angewendet, bei dem die Dicarbonsäure und das Diol in einem Reaktor mit oder ohne Katalysator verestert und das bei der Veresterung entstehende Wasser bei vermindertem Druck mit oder ohne Schleppmittel aus dem Reaktionsgemisch entfernt wird. Solche Oligoester sind erfindungsgemäß einsetzbar, so daß der Einsatz mehrerer Reaktoren hintereinander, wie man sie üblicherweise in quasikontinuierlichen Reaktionskaskaden benutzt, nicht mehr notwendig ist.

3

Als Temperaturbereich kommt der Bereich von 100 bis 240°C und als Druckbereich der von 100 bis 1500 mbar in Frage. Diese Durchführung in einer Veresterungsstufe vermag alleine jedoch kaum die relativ langen Reaktionszeiten von beispielsweise 12 Stunden bis zu einer vollständigen Veresterung und mehr wesentlich zu verkürzen, wenn nicht durch technische Maßnahmen, beispielsweise durch ein eingebautes schnellaufendes Rührsystem, die Wasseraustragung aus dem Reaktionsgemisch beschleunigt wird. Eine weitere Beschleunigung der Wasseraustragung ist beispielsweise durch Einblasen eines Treibgases, beispielsweise durch Einblasen von Stickstoff, möglich.

Bei einstufiger Fahrweise kann die Oligoveresterung jedoch auch so geführt werden, daß in einem gewissen Zeittakt mehrere Reaktoren zeitlich versetzt nebeneinander mit Veresterungsgemisch gefüllt werden. So steht jeweils nach kürzerer Zeit, als für die gesamte Veresterung benötigt wird, eine neue Charge Oligoester für die folgende Hydrierung zur Verfügung.

Die für die Oligoveresterung in Frage kommenden Reaktionsapparate bestehen aus säurefestem Material und sind mit einem wirksamen Rührer und einer aufgesetzten Destillationskolonne üblicher Bauart mit 8 bis 15 Böden ausgerüstet.

Verestert man beispielsweise eine Dicarbonsäure unter den angegebenen Veresterungsbedingungen mit einem Diol im molaren Verhältnis von 1:1, so erhält man ein Gemisch von Oligoestern unterschiedlicher Oligomerisierungsgrade (= Zahl der Moleküle Dicarbonsäure im Oligoester), deren Molmassenverteilung recht genau einer Glokkenkurve folgt, deren mittlerer Oligomerisierungsgrad n im Sinne des obigen Reaktionsschemas bei n = 7 liegt. Die Säurezahl liegt oberhalb von 50 mg KOH/g Reaktionsprodukt. Zur Verschiebung der mittleren Kettenlänge des Oligoesters zu kürzeren Kettenlängen ist es zweckmäßig, in Gegenwart eines molaren Überschusses an Diol zu verestern. Verestert man nun die Dicarbonsäure unter den angegebenen Reaktionsbedingungen mit einem Diol im molaren Verhältnis von 1:2, so hat man zwar die Gewißheit, daß die freien Carboxylgruppen relativ schnell verestert sind und das erhaltene Oligoestergemisch nur noch eine mittlere Kettenlänge von beispielsweise n = 5 hat, muß aber einen relativ hohen Anteil an Diol einsetzen. Es wurde nun gefunden, daß man auch schon bei einem geringen molaren Überschuß an Diol, bezogen auf die Dicarbonsäure unter Vermeidung längerer Reaktionszeiten, bei höheren Säurezahlen als 40 mg KOH/g Reaktionsprodukt Oligoester mit einer mittleren Kettenlänge von 4 herstellen kann. Das molare Verhältnis für den benötigten Oligoester beträgt 1,0 bis 1,15 mol, bevorzugt 1,05 bis 1,10 mol Diol pro 1 mol der Dicarbonsäure. Der hierbei erhaltene mittlere Veresterungsgrad (Oligomerisierungsgrad) liegt im Bereich von n = 3 bis n = 4,5, bevorzugt n = 4.

Die eingesetzten Dicarbonsäuren und die eingesetzten Diole haben üblicherweise eine Reinheit von mehr als 99 %. Dadurch, daß auf systemfremde Stoffe verzichtet werden kann, reicht jedoch auch die Reinheit von Destillationsrückläufen aus dem der Oligoveresterung folgenden Hydrierschritt im Rahmen des erfindungsgemäßen Verfahrens völlig aus, selbst wenn diese Destillationsrückläufe geringe Mengen nicht hydrierten Oligoesters enthalten. Solche zurückgeführten Oligoester und andere Anteile sind bei der Festlegung des genannten Molverhältnisses zwischen Diol und Dicarbonsäure zu berücksichtigen.

Es ist ein weiterer Vorteil des erfindungsgemäßen Verfahrens, daß der so hergestellte Oligoester ohne zusätzliche Reinigungsschritte direkt in der Hydrierstufe weiterverarbeitet werden kann.

Der Hydrierschritt im erfindungsgemäßen Verfahren erfolgt in der flüssigen Phase mit überschüssigem Wasserstoff, der mindestens das 20- bis 100-fache der stöchiometrisch nötigen Menge beträgt. Durch das Arbeiten in der flüssigen Phase wird der Energieaufwand für Gasphasenprozesse kostensparend verringert.

Während zur Hydrierung von Estern noch vielfach ein diskontinuierliches Verfahren (Batch-Prozeß) angewendet wird, bei welchem pulverförmige Katalysatoren in einem Suspensionsverfahren zum Einsatz kommen, kann das erfindungsgemäße Verfahren in seiner Hydrierstufe vollkontinuierlich betrieben werden. Weiterhin wird die Hydrierstufe an einem stückigen Katalysator durchgeführt. Hierdurch werden die Schwierigkeiten mit pulverförmigen Katalysatoren umgangen, nämlich die Schwierigkeit, Pulverkatalysatoren gezielt und gleichmäßig zu aktivieren, die Schwierigkeit, Pulverkatalysatoren mit Hilfe von speziellen Schlammpumpen umzuwälzen, und die Schwierigkeit, Pulverkatalysatoren vom Reaktionsprodukt quantitativ abzutrennen. Schlammpumpen unterliegen nämlich einer hohen mechanischen Beanspruchung.

Die quantitative Entfernung pulverförmiger Katalysatoren ist weiterhin aufwendig, weil sie eine Grob- und eine Feinfiltration mit Apparaten in Wechselausführung erfordert. Ferner ist die Gefahr groß, daß die Katalysatoren durch diese zusätzlichen Operationen schnell ihre Aktivität verlieren und daher weiterhin hohe Katalysatorverbräuche in Kauf zu nehmen sind. Im Gegensatz zu diesen aufgezeigten Schwierigkeiten besitzen die erfindungsgemäß einzusetzenden neuen stückigen Katalysatoren eine hohe Säureunempfindlichkeit, eine hohe Druckunempfindlichkeit und weisen eine hohe Aktivität auf, die auch über einen Zeitraum von ein bis mehreren Jahren nicht nachläßt. Der letztere Vorteil ist sehr wichtig, da auch bei stückigen, im Festbett angeordneten Katalysatoren ein häufiger Katalysatorwechsel sehr aufwendig ist.

Der erfindungsgemäß einzusetzende stückige Katalysator besteht aus verpreßten Pulvern von Cu-, Mn- und Al-Oxiden, bei denen der Cu-Anteil 30,0 bis 55,0 Gew.-%, der Mn-Anteil 3,0 bis 15,0 Gew.-% und der Al-Anteil 10,0 bis 25,0 Gew.-%, jeweils gerechnet als Metall, beträgt, wobei alle Angaben auf die Gesamtmenge des Oxidpulvergemisches bezogen sind und der Rest zu 100 Gew.-% Sauerstoff darstellt. Ein solcher Katalysator kann in dieser Form ohne weitere Zusätze zum erfindungsgemäßen Hydrierschritt eingesetzt werden. In vorteilhafter Weise enthält er jedoch zusätzlich einen Gehalt von mindestens einem Oxid von Metallen der VI. Nebengruppe des Periodensystems der Elemente (Mendelejew), insbesondere Cr, Mo, W, sowie gegebenenfalls einem Anteil einer Erdalkaliverbindung wie Mg(OH)$_2$ oder MgO, Ca(OH)$_2$ oder CaO, Sr(OH)$_2$ oder SrO oder Ba(OH)$_2$ oder BaO mit einem Erdalkalimetall-Anteil von 0,5 bis 7,0 Gew.-%, gerechnet als Metall und bezogen auf das Gesamtgewicht des Katalysators.

Als Elemente der VI. Nebengruppe kommen hierbei Chrom, Molybdän und Wolfram in Frage. Oxide der Elemente Chrom, Molybdän und Wolfram, bevorzugt Chrom und Molybdän, können sowohl einzeln als auch im Gemisch von Oxiden mehrerer der genannten Elemente eingesetzt werden. Die Gesamtmenge von Oxiden der VI. Nebengruppe des Periodensystems im verpreßten Oxidpulver beträgt 0,05 bis 3,0 Gew.-%, bevorzugt 0,1 bis 0,8 Gew.-%, gerechnet als Metall und bezogen auf die Menge des gesamten Oxidpulvers für die Hydrierkatalysatoren. Für den Fall des Einsatzes mehrerer Oxide von Elementen der VI. Nebengruppe des Periodensystems liegt jedes dieser gemischten Oxide in einer Menge vor, die nicht kleiner als 20 % und nicht größer als 80 % des genannten Gesamtbereichs von 0,05 bis 3,0 Gew.-% ist.

Die Herstellung der trägerfreien stückigen Katalysatoren kann nach gebräuchlichen Methoden durch Verpressen der Metalloxidpulver, beispielsweise auf Tablettier- oder Pelletiermaschinen, unter hohem Druck erfolgen, wobei zur Verbesserung des Haftvermögens der Metalloxidpartikel auch Graphit und/oder Klebstoffe in Mengen von 0,5 bis 3,0 Gew.-%, bezogen auf das Gesamtgewicht der zu verpressenden Bestandteile, zum Einsatz kommen können. Beispiele für die stückige Form der Katalysatoren sind Tabletten, Kugeln oder Granulate mit Abmessung von 2 bis 10 mm, bevorzugt 3 bis 7 mm. Tablettierte Formkörper können weiterhin zur Vergrößerung der äußeren Oberfläche mit einer axialen Durchbohrung versehen sein. Solche Formkörper haben makroskopisch betrachtet eine glatte Oberfläche. Um eine hohe Standfestigkeit (Lebensdauer) zu erreichen, müssen die stückigen Katalysatoren eine Druckfestigkeit von 50 bis 200 N, bevorzugt 70 bis 170 N, auf die Formkörperoberfläche aufweisen. Die stückigen Katalysatoren besitzen ferner eine innere Oberfläche von 10 bis 90 m$^2$/g, bevorzugt 30 bis 80 m$^2$/g. Die Druckfestigkeit der trägerfreien stückigen Katalysatoren kann nach DIN 50 106 bestimmt werden. Die Bestimmung der inneren Oberflächen erfolgt nach Analyt. Chem. 30 (1958), 1387 bzw. nach Adsorption, Surface Area and Porosity, London 1967, Kap. 2 und 6.

Die als Hydrierkatalysatoren eingesetzten Formkörper aus verpreßten Pulvern von Cu-, Mn- und Al-Oxiden mit einem Gehalt von mindestens einem Oxid von Metallen der VI. Nebengruppe des Periodensystems müssen vor ihrem Einsatz sorgfältig reduziert werden. Dies geschieht durch eine Behandlung mit Wasserstoff bei 180 bis 280°C, wobei zu Beginn der Behandlung ein Gemisch aus 10 bis 15 Vol.-% H$_2$ und 90 bis 85 Vol.-% Inertgas (z.B. Stickstoff) eingesetzt wird und im Verlaufe der Behandlung der Inertgasanteil bis auf Null Vol.-% vermindert wird. Eine solche Behandlung wird in einem Zeitraum von ca. 12 bis 24 Stunden durchgeführt und ist beendet, wenn der Katalysator keinen Wasserstoff mehr verbraucht und infolgedessen kein Reaktionswasser mehr bildet.

Der Hydrierungsschritt im erfindungsgemäßen Verfahren wird bevorzugt kontinuierlich in der flüssigen Phase bei 180 bis 250°C, bevorzugt bei 190 bis 240°C und einem H$_2$-Druck von 100 bis 400 bar, bevorzugt 150 bis 300 bar, mit reinem Wasserstoff durchgeführt.

Während es prinzipiell gleich ist, das zu hydrierende Oligoestergemisch im Hydrierreaktor von unten nach oben oder von oben nach unten strömen zu lassen, hat es sich als vorteilhaft herausgestellt, das Oligoestergemisch von oben nach unten über den Katalysator fließen zu lassen (Rieselphase). Dabei kann das zu hydrierende Oligoestergemisch entweder gemeinsam mit dem separat eingeführten oder vorher zugemischten Wasserstoff über den Katalysator strömen (Gleichstromverfahren) oder aber dem Wasserstoff entgegengeführt werden (Gegenstromverfahren).

Der Hydrierreaktor kann entweder ein einzelnes Hochdruckrohr aus Stahl oder einer Stahllegierung sein, das mit dem Katalysator ganz oder teilweise gefüllt wird, wobei bei größeren Rohrquerschnitten auch die Anwendung der stückigen trägerfreien Katalysatoren auf Horden (Drahtkörbe oder ähnliches) nützlich sein kann; man kann jedoch auch Hochdruckrohrbündel innerhalb eines gemeinsamen Mantels anwenden, wobei die Einzelrohre wiederum mit den trägerfreien stückigen Katalysatoren ganz oder teilweise gefüllt werden.

Die stündliche Katalysatorbelastung kann bei 200 bis 600 ml Oligoestergemisch pro Liter Katalysator liegen. Unter den genannten Reaktionsbedingungen sind hohe Katalysatorstandzeiten von 8 000 bis 16 000 Stunden zu erreichen. Das den Hydrierreaktor verlassende Reaktionsgemisch besteht nach der Entspannung, bei welcher man den überschüssigen Wasserstoff abfangen und nach erfolgter Kompression und Ergänzung des verbrauchten Wasserstoffs erneut zum Einsatz bringen kann, zu 97,5 Gew.-% und mehr aus dem zu erwartenden Diol oder dem zu erwartenden Gemisch von Diolen. Es enthält an organischen Leichtsiedern maximal 2,0 Gew.-% sowie an Hochsiedern maximal 1,5 Gew.-% eines höhermolekularen Rückstandes. Die Höhersieder stellen im wesentlichen nicht umgesetztes Oligoestergemisch dar und können in den Prozeß zurückgeführt werden, so daß dessen Gesamtselektivität bezüglich der Diole bei mindestens 98,0 Gew.-% liegt. Das erzeugte Diol wird nach der destillativen Entfernung von Leicht- und Hochsiedern in einer Reinheit von mindestens 99,9 Gew.-% erhalten und ist in dieser Reinheit für alle weiterverarbeitenden Prozesse einsetzbar.

## Beispiele

## Beispiel 1

In einen Reaktionsapparat aus säurefestem Material mit einem Volumen von 5 l, der mit einem Schnellrührsystem üblicher Bauart (Turbinenrührer, Drehzahl: 800 bis 1200/min) und einer Destillationskolonne (10 theoret. Böden) versehen war, wurde eine 100°C warme Lösung von 730,7 g (5 mol) Adipinsäure in 620 g (5,25 mol) Hexandiol-1,6 gegeben, unter Rühren schnell auf eine Reaktionstemperatur von 140 bis 170°C erhitzt und das sich bildende Reaktionswasser bei Normaldruck abdestilliert. Nach 1,5 Stunden Verweilzeit wurde schließlich bei einem Druck von 400 mbar und bei einer Reaktionstemperatur von 170 bis 190°C der Rest des Reaktionswassers entfernt. Nach weiteren 2,0 Stunden Verweilzeit (insgesamt 3,5 Stunden) war der gewünschte Veresterungsgrad erreicht. Das erhaltene oligomere Hexandiol-1,6-adipinat (1 265 g) hatte einen mittleren Grad der Oligoveresterung (= Zahl der Moleküle Adipinsäure im Oligoester) von n = 4 (gemessen durch Gelpermeabilitätschromatographie) und eine Säurezahl von 50 mg KOH/g Reaktionsgemisch.

## Beispiel 2

Ein senkrecht stehendes, wärmeisoliertes Hochdruckrohr aus nichtrostendem säurefestem Metall von 45 mm Innendurchmesser und 1 m Länge, das vorher mit Stickstoff sauerstofffrei gespült worden war, wurde mit 1,4 l eines durch Tablettierung von Pulvern von Kupfer-, Mangan-, Aluminium- und Chromoxiden hergestellten Hydrierungskatalysators gefüllt. Der Kupfergehalt der Tabletten lag bei 41 Gew.-%, der Mangangehalt bei 6,6 Gew.-%, der Aluminiumgehalt bei 19,0 Gew.-% und der Chromgehalt bei 0,46 Gew.-%. Die Tabletten hatten bei einer Zylinderhöhe von 3 mm und einem Durchmesser von 3 mm eine Druckfestigkeit von 100 N auf die Zylindermantelfläche und eine innere Oberfläche von 45 m$^2$/g. Zur Aktivierung des eine Mischung von Metalloxiden enthaltenden Katalysators wurden die Tabletten zunächst 6 Stunden im Stickstoffstrom getrocknet (Temperatur: max. 200°C, Menge: 5 Nm$^3$N$_2$/h). Die eigentliche Aktivierung erfolgte bei einem Stickstoffdruck von 200 bar und einer Temperatur zwischen 180 und 280°C, wobei dem Inertgas allmählich Wasserstoff zugemischt wurde, dessen Zumischungsanteil in der Anfangsphase 10 bis 15 Vol.-% nicht übersteigen durfte. Innerhalb von 24 Stunden wurde der Stickstoffanteil der Gasmischung mehr und mehr vermindert, bis schließlich reiner Wasserstoff den Reaktor durchströmte. Die Reaktion war beendet, wenn kein Reaktionswasser, das in einem nachgeschalteten Abscheider gesammelt wurde, mehr gebildet wurde. Nach der Aktivierung des Hydrierkatalysators wurde der Wasserstoffdruck im Reaktorsystem auf 300 bar erhöht. Anschließend wurden stündlich 300 g Hexandiol-1,6-adipinat nach Beispiel 1 gemeinsam mit 5 Nm$^3$ Wasserstoff bei einem Druck von 300 bar durch das Hochdruckrohr gepumpt, wobei das Hexandiol-1,6-adipinat vor Eintritt in das Hochdruckrohr in einem vorgeschalteten elektrisch beheizten Wärmeaustauscher auf eine Temperatur von 230°C erhitzt wurde. Das das Reaktionsrohr verlassende Reaktionsprodukt (Roh-Hexandiol-1,6) wurde in einem zweiten Wärmeaustauscher (Wasserkühler) bei 300 bar Wasserstoffdruck auf eine Temperatur <60°C abgekühlt und in einem Gasabscheider von überschüssigen Wasserstoff, der wieder in das Hydriersystem zurückgeführt wurde, getrennt. Nach weiterer Abkühlung auf eine Temperatur <30°C und Entspannung auf Normaldruck wurde das Reaktionsprodukt gaschromatographisch untersucht. Es enthielt an organischen Leichtsiedern lediglich 1,8 Gew.-% Monoalkohole der C-Zahlen 1 bis 6 sowie an Höhersiedern 1,1 Gew.-% nicht umgesetztes Hexandiol-1,6-adipinat, so daß der Hexandiol-1,6-Gehalt des organischen Reaktionsproduktes bei 97,1 Gew.-% lag. Das erzeugte Hexandiol-1,6 wurde nach der destillativen Entfernung von Leicht- und Hochsiedern in einer Reinheit von ≥99,9 Gew.-% erhalten. Da die Höhersieder wieder in den Prozeß zurückgeführt werden konnten, lag dessen Gesamtselektivität bezüglich Hexandiol-1,6 bei 98,2 Gew.-%. Der Katalysator war nach einer Laufzeit von 3 000 Stunden unverändert wirksam, so daß sich die Zusammensetzung des Reaktionsproduktes über diesen Zeitraum nicht veränderte.

## Beispiel 3

Ein Hochdruckrohr wie in Beispiel 2 wurde unter Inertgas mit 1,4 l eines durch Tablettierung von Pulvern von Kupfer-, Mangan-, Aluminium-, Chrom- und Molybdänoxiden hergestellten Hydrierungskatalysators gefüllt. Der Kupfergehalt der Tabletten lag bei 42,0 Gew.-%, der Mangangehalt bei 7,0 Gew.-%, der Aluminiumgehalt bei 18,5 Gew.-%, der Chromgehalt bei 0,48 Gew.-% und der Molybdängehalt bei 0,46 Gew.-%. Die Tabletten hatten bei einer Zylinderhöhe von 5 mm und einem Durchmesser von 5 mm eine Druckfestigkeit von 105 N auf die Zylindermantelfläche und eine innere Oberfläche von 48 m$^2$/g. Nach der Aktivierung des Hydrierkatalysators wie in Beispiel 2 wurde der Wasserstoffdruck auf 300 bar erhöht. Anschließend wurden stündlich 450 g Hexandiol-1,6-adipinat nach Beispiel 1 gemeinsam mit 5 Nm$^3$ Wasserstoff bei einem Druck von 300 bar kontinuierlich durch das Hochdruckrohr gepumpt, wobei das Hexandiol-1,6-adipinat vor Eintritt in das Hochdruckrohr auf eine Temperatur von 225°C erhitzt wurde. Das Reaktionsprodukt (RohHexandiol-1,6) enthielt nach der Abtrennung von überschüssigem Wasserstoff und der Abkühlung auf eine

6

Temperatur <30°C laut gaschromatographischer Analyse an organischen Leichtsiedern 0,9 Gew.-% Monoalkohole der C-Zahlen 1 bis 6 sowie an Höhersiedern 0,8 Gew.-% nicht umgesetztes Hexandiol-1,6-adipinat, so daß der Hexandiol 1,6-Gehalt bei 98,3 Gew.-% lag. Nach der destillativen Entfernung von Leicht- und Höhersiedern wurde das erzeugte Hexandiol-1,6 in einer Reinheit von 99,9 Gew.-% erhalten. Da die Höhersieder wieder in den Prozeß zurückgeführt werden konnten, lag dessen Gesamtselektivität bezüglich Hexandiol-1,6 bei 99,1 Gew.-%. Der Katalysator war nach einer Laufzeit von 3500 Stunden unverändert wirksam, so daß sich die Zusammensetzung des Reaktionsproduktes über diesen Zeitraum nicht veränderte.

**Beispiel 4**

Ein Hochdruckrohr wie in Beispiel 2 wurde unter Inertgas mit 1,4 l eines durch Tablettierung von Pulvern von Kupfer-, Mangan-, Aluminium-, Chrom- und Wolframoxiden hergestellten Hydrierungskatalysators gefüllt. Der Kupfergehalt der Tabletten lag bei 42,0 Gew.-%, der Mangangehalt bei 7,0 Gew.-%, der Alumimumgehalt bei 18,5 Gew.-%, der Chromgehalt bei 0,48 Gew.-% und der Wolframgehalt bei 0,44 Gew.-%. Die Tabletten hatten bei einer Zylinderhöhe von 5 mm und einem Durchmesser von 5 mm eine Druckfestigkeit von 102 N auf die Zylindermantelfläche und eine innere Oberfläche von 52 m$^2$/g. Nach der Aktivierung des Hydrierkatalysators wie in Beispiel 2 wurde der Wasserstoffdruck auf 300 bar erhöht. Anschließend wurden stündlich 250 g Hexandiol-1,6-adipinat nach Beispiel 1 gemeinsam mit 5 Nm$^3$ Wasserstoff bei einem Druck von 300 bar kontinuierlich durch das Hochdruckrohr gepumpt, wobei das Hexandiol-1,6-adipinat vor Eintritt in das Hochdruckrohr auf eine Temperatur von 225°C erhitzt wurde. Das Reaktionsprodukt (RohHexandiol-1,6) enthielt nach der Abtrennung von überschüssigem Wasserstoff und der Abkühlung auf eine Temperatur <30°C laut gaschromatographischer Analyse an organischen Leichtsiedern 0,9 Gew.-% Monoalkohole der C-Zahlen 1 bis 6 sowie an Höhersiedern 1,8 Gew.-% nicht umgesetztes Hexandiol-1,6-adipinat, so daß der Hexandiol-1,6-Gehalt des organischen Reaktionsproduktes bei 97,3 Gew.-% lag. Nach der destillativen Entfernung von Leicht- und Höhersiedern wurde das erzeugte Hexandiol-1,6 in einer Reinheit von 99,9 Gew.-% erhalten. Da die Höhersieder wieder in den Prozeß zurückgeführt werden konnten, lag dessen Gesamtselektivität bezüglich Hexandiol-1,6 bei 99,1 Gew.-%. Der Katalysator war nach einer Laufzeit von 700 Stunden unverändert wirksam, so daß sich die Zusammensetzung des Reaktionsproduktes über diesen Zeitraum nicht veränderte.

**Beispiel 5**

In einen Reaktionsapparat aus säurefestem Material mit einem Volumen von 5 l, der mit einem Schnellrührsystem üblicher Bauart (Turbinenrührer, Drehzahl: 800 bis 1 200/min) und einer Destillationskolonne (10 theoret. Böden) versehen war, wurde eine 100°C warme Lösung von 590,5 g (5 mol) Bernsteinsäure in 602 g (5,1 mol) Butandiol-1,4 gegeben, unter Rühren schnell auf eine Reaktionstemperatur von 120 bis 130°C erhitzt und das sich bildende Reaktionswasser bei Normaldruck abdestilliert. Nach 1,5 Stunden Verweilzeit wurde schließlich bei einem Druck von 400 mbar und bei einer Reaktionstemperatur von 130 bis 160°C der Rest des Reaktionswassers entfernt. Nach weiteren 2,0 Stunden Verweilzeit (insgesamt 3,5 Stunden) war der gewünschte Veresterungsgrad erreicht. Das erhaltene oligomere Butandiol 1,4-succinat (1.098 g) hatte einen mittleren Grad der Oligoveresterung (= Zahl der Moleküle Bernsteinsäure im Oligoester) von n = 4 (gemessen durch Gelpermeabilitätschromatographie) und eine Säurezahl von 58 mg KOH/g Reaktionsgemisch.

**Beispiel 6**

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l eines durch Tablettierung von Pulvern von Kupfer-, Mangan-, Aluminium- und Chromoxiden hergestellten Hydrierungskatalysators gefüllt. Der Kupfergehalt der Tabletten lag bei 41 Gew.-%, der Mangangehalt bei 6,6 Gew.-%, der Aluminiumgehalt bei 19,0 Gew.-% und der Chromgehalt bei 0,46 Gew.-%. Die Tabletten hatten bei einer Zylinderhöhe von 3 mm und einem Durchmesser von 3 mm eine Druckfestigkeit von 100 N auf die Zylindermantelfläche und eine innere Oberfläche von 45 m$^2$/g. Zur Aktivierung des Katalysators wurden die Tabletten zunächst 6 Stunden im Stickstoffstrom getrocknet (Temperatur: max. 200°C, Menge: 5 Nm$^3$N$_2$/h). Die eigentliche Aktivierung erfolgte bei einem Stickstoffdruck von 200 bar und einer Temperatur zwischen 180 und 280°C, wobei dem Inertgas allmählich Wasserstoff zugemischt wurde, dessen Zumischungsanteil in der Anfangsphase 10 bis 15 Vol.-% nicht übersteigen durfte. Innerhalb von 24 Stunden wurde der Stickstoffanteil der Gasmischung mehr und mehr vermindert, bis schließlich reiner Wasserstoff den Reaktor durchströmte. Die Reaktion war beendet, wenn kein Reaktionswasser, das in einem nachgeschalteten Abscheider gesammelt wurde, mehr gebildet wurde. Nach der Aktivierung des Hydrierkatalysators wurde der Wasserstoffdruck im Reaktorsystem auf 300 bar erhöht. Anschließend wurden stündlich 350 g Butandiol-1,4-succinat nach Beispiel 5 gemeinsam mit 5 Nm$^3$ Wasserstoff bei einem Druck von 300 bar durch das Hochdruckrohr gepumpt, wobei das Butandiol-1,4-succinat vor Eintritt in das Hochdruckrohr in einem vorgeschalteten elektrisch beheizten Wärmeaustauscher auf eine Temperatur von 230°C erhitzt wurde. Das Reaktionsprodukt (Roh-Butandiol-1,4) wurde in einem zweiten Wärmeautauscher (Wasserkühler) bei 300 bar Wasserstoffdruck auf eine Temperatur <60°C abgekühlt und in einem Gasabscheider vom überschüssigen Wasserstoff, der wieder in das Hydriersystem zurückgeführt wurde, getrennt. Nach weiterer Abkühlung auf eine Temperatur <30°C und Entspannung auf Normaldruck wurde das Reaktionsprodukt gaschromatographisch untersucht. Es enthielt an organischen Leichtsiedern lediglich 1,0 Gew.-% Tetrahydrofuran und 0,9 Gew.-% Monoalkohole der C-Zahlen 1 bis 4 sowie an Höhersiedern 1,1 Gew.-% nicht umgesetztes Butandiol-1,4-succinat, so daß der Butandiol-1,4-Gehalt des organischen Reaktionsproduktes bei 97,0 Gew.-% lag. Das erzeugte Butandiol-1,4 wurde nach der destillativen Entfernung von Leicht- und Hochsiedern in einer Reinheit von $\geq$99,9 Gew.-% erhalten. Da die Höhersieder wieder in den Prozeß zurückgeführt werden konnten, lag dessen Gesamtselektivität bezüglich Butandiol-1,4 bei 98,1 Gew.-%.

**Patentansprüche**

1. Verfahren zur Herstellung von aliphatischen $\alpha,\omega$-Diolen mit 4 bis 12 C-Atomen aus aliphatischen $\alpha,\omega$-Dicarbonsäuren mit 4 bis 12 C-Atomen durch Oligoveresterung der Dicarbonsäuren mit den Diolen zu einem Diol-Dicarboxylat der mittleren Kettenlänge n = 3 bis n = 4,5 und Säurezahlen von 40 bis 60 mg KOH/g Reaktionsgemisch und katalytische Hydrierung des entstandenen Oligoesters in der Flüssigphase, dadurch gekennzeichnet, daß der Oligoester kontinuierlich bei 180 bis 250°C, bevorzugt bei 190 bis 240°C, und einem $H_2$-Druck von 100 bis 400 bar, bevorzugt von 150 bis 300 bar, wobei die $H_2$-Menge das 20- bis 100-fache der stöchiometrisch benötigten Menge beträgt, an einem Zn-Oxid-freien stückigen Katalysator, bestehend aus verpreßten Pulvern von Cu-, Mn- und Al-Oxiden mit einem Gehalt von mindestens einem Oxid von Metallen der VI. Nebengruppe des Periodensystems der Elemente (Mendelejew) hydriert wird.

2. Verfahren nach Anspruch 1, dadurch gekennzeichnet, daß die Oligoveresterung in einem Veresterungsschritt unter destillativer Entfernung des Reaktionswassers im Temperaturbereich von 100 bis 240°C und im Druckbereich von 100 bis 1 500 mbar durchgeführt wird.

3. Verfahren nach Ansprüchen 1 und 2, dadurch gekennzeichnet, daß der eingesetzte Oligoester aus einem Gemisch von 1,0 bis 1,15 mol, bevorzugt 1,05 bis 1,10 mol Diol pro 1 mol Dicarbonsäure so hergestellt wird, daß ein durchschnittlicher Veresterungsgrad von 4 erreicht wird.

4. Verfahren nach Ansprüchen 1 bis 3, dadurch gekennzeichnet, daß der eingesetzte Oligoester ohne weitere Reinigung zur Hydrierung eingesetzt wird.

5. Verfahren nach Ansprüchen 1 bis 4, dadurch gekennzeichnet, daß im Gemisch der verpreßten Oxidpulver der Cu-Anteil 30,0 bis 55,0 Gew.-%, der Mn-Anteil 3,0 bis 15,0 Gew.-% und der Al-Anteil 10,0 bis 25,0 Gew.-%, der Gehalt an Oxiden der VI. Nebengruppe 0,05 bis 3,0 Gew.-% beträgt, wobei alle Angaben als Metalle berechnet und auf die Gesamtmenge des Oxidpulvergemisches bezogen sind und der Rest zu 100 Gew.-% Sauerstoff darstellt.

6. Verfahren nach Ansprüchen 1 bis 5, dadurch gekennzeichnet, daß eines oder mehrere Oxide der VI. Nebengruppe des Periodensystems der Elemente im verpreßten Oxidpulver vorliegen und deren Gesamtmenge 0,1 bis 0,8 Gew.-% des gesamten Oxidpulvers beträgt.

7. Verfahren nach Ansprüchen 1 bis 6, dadurch gekennzeichnet, daß der Katalysator zusätzlich einen Gehalt an 0,5 bis 7,0 Gew.-% einer Erdalkaliverbindung, gerechnet als Metall und bezogen auf die Menge des gesamten Oxidpulvers, aufweist.

8. Verfahren nach Ansprüchen 1 bis 7, dadurch gekennzeichnet, daß der stückige Katalysator aus verpreßten Oxidpulvem Zylinder- oder Kugelform mit Abmessungen von 2 bis 10 mm, bevorzugt 3 bis 7 mm hat und eine Druckfestigkeit von 50 bis 200 N, bevorzugt 70 bis 170 N, auf die Formkörperoberfläche und eine innere Oberfläche von 10 bis 90 $m^2$/g, bevorzugt 30 bis 80 $m^2$/g aufweist.

9. Verfahren nach Ansprüchen 1 bis 8, dadurch gekennzeichnet, daß der Katalysator aus verpreßtem Oxidpulver vor der Hydrierung durch eine Behandlung mit $H_2$ bei 180 bis 280°C aktiviert wird, wobei zu Beginn der Behandlung ein Gemisch aus 10 bis 15 Vol.-% $H_2$ und 90 bis 85 Vol.-% Inertgas eingesetzt wird und im Verlaufe der Behandlung der Inertgasanteil bis auf Null Vol.-% vermindert wird.

Europäisches
Patentamt

**EUROPÄISCHER RECHERCHENBERICHT**

Nummer der Anmeldung

EP 98 12 2726

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,A | EP 0 721 928 A (BAYER AG) 17. Juli 1996<br>----- | 1 | C07C29/149<br>B01J23/76 |

**RECHERCHIERTE SACHGEBIETE (Int.Cl.6)**

C07C
B01J

Der vorliegende Recherchenbericht wurde für alle Patentansprüche erstellt

| Recherchenort | Abschlußdatum der Recherche | Prüfer |
|---|---|---|
| MÜNCHEN | 16. März 1999 | Goetz, G |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 98 12 2726

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-03-1999

| Im Recherchenbericht angeführtes Patentdokument | Datum der Veröffentlichung | Mitglied(er) der Patentfamilie | | Datum der Veröffentlichung |
|---|---|---|---|---|
| EP 0721928 A | 17-07-1996 | DE | 19500783 A | 18-07-1996 |
| | | JP | 9020704 A | 21-01-1997 |
| | | US | 5696303 A | 09-12-1997 |

EPO FORM P0461

Für nähere Einzelheiten zu diesem Anhang : siehe Amtsblatt des Europäischen Patentamts, Nr.12/82